# EUROPÄISCHE PATENTSCHRIFT

(11) **EP 3 503 935 B1**
(45) Veröffentlichungstag und Bekanntmachung des Hinweises auf die Patenterteilung: **24.08.2022**
(21) Anmeldenummer: 17732924.0
(22) Anmeldetag: 27.06.2017
(51) Int. Cl.: A61M 1/00

(54) **VORRICHTUNG ZUR UNTERDRUCKBEHANDLUNG VON WUNDEN AM MENSCHLICHEN KÖRPER**
DEVICE FOR THE VACUUM TREATMENT OF WOUNDS ON THE HUMAN BODY
DISPOSITIF DE TRAITEMENT PAR PRESSION NÉGATIVE DE PLAIES SUR LE CORPS HUMAIN

(30) Priorität: 25.08.2016 DE 102016115836
(43) Veröffentlichungstag der Anmeldung: 03.07.2019
(73) Patentinhaber: Paul Hartmann AG, 89522 Heidenheim (DE)
(72) Erfinder: HOFSTETTER, Juergen, 52349 Düren (DE); BEYRLE, Karina, 89075 Ulm (DE); KRäMER, Maja, 89542 Herbrechtingen (DE); ECKSTEIN, Axel, 89522 Heidenheim (DE); WAIBEL, Tabea, 89168 Niederstotzingen (DE); KLEIN, Peter, 71254 Ditzingen (DE); MURGULESCU, Mihai, 90411 Nuernberg (DE)
(74) Vertreter: DREISS Patentanwälte PartG mbB
(86) Internationale Anmeldenummer: PCT/EP2017/065846
(87) Internationale Veröffentlichungsnummer: WO 2018/036691

(56) Entgegenhaltungen:
- WO-A2-01/37922
- WO-A2-2014/163733
- DE-A1-102010 060 543
- US-A1- 2013 066 301
- US-A1- 2016 038 658

## Beschreibung

Die Erfindung betrifft eine Vorrichtung zur Bereitstellung von Unterdruck zur Unterdruckbehandlung von Wunden am menschlichen Körper, mit einer Unterdruck erzeugenden Einrichtung und einem nach Gebrauch wegwerfbaren Einweg-Behälter zur Aufnahme von Flüssigkeiten, insbesondere von aus einer Wunde abgesaugten Wundsekreten, mit einem Anschluss für eine zum Körper führende Saugleitung, wobei die Unterdruck erzeugende Einrichtung in einem ersten Gehäuseteil der Vorrichtung angeordnet ist und der Behälter von einem zweiten Gehäuseteil der Vorrichtung gebildet ist, wobei der erste und der zweite Gehäuseteil lösbar gegeneinander fixierbar sind und wobei der Behälter seinerseits wenigstens ein erstes und ein zweites Wandungsteil umfasst, welche unlösbar miteinander verbunden sind und dabei das Innere des Behälters begrenzen.

Bei derartigen Vorrichtungen zur Unterdruckbehandlung von Wunden kommuniziert die Unterdruck erzeugende Einrichtung über den Behälter und über die Saugleitung mit der Wunde oder der Wundumgebung, wobei ein luftundurchlässiges Abdeckmaterial als Unterdruckwundverband zum luftdichten Verschließen der Wunde und der Wundumgebung vorgesehen ist, so dass ein Unterdruck im Wundraum herstellbar ist und Flüssigkeiten aus dem Wundraum in den Behälter absaugbar sind.

Der Begriff eines Unterdrucks bezeichnet im Zusammenhang mit der vorliegenden Erfindung einen gegenüber dem Umgebungsluftdruck (atmosphärischer Luftdruck) erniedrigten Luftdruck insbesondere innerhalb eines Wundverbands. Das Abdeckmaterial eines Wundverbands zum luftdichten Verschließen eines Wundraums muss daher so ausgebildet sein, dass es der sich einstellenden Druckdifferenz standzuhalten vermag, damit der Unterdruck im Wundraum überhaupt angelegt und aufrechterhalten werden kann.

Im Bereich der Unterdrucktherapie in der Wundbehandlung wird der Unterdruck quantitativ als Druckdifferenz zwischen dem Umgebungsluftdruck und dem unterhalb des Abdeckmaterials angelegten Luftdruck angegeben. Typischerweise beträgt diese Druckdifferenz im Bereich der Unterdrucktherapie höchstens 250 mm Hg (mm Quecksilbersäule) (1 mm Hg = 1 Torr. = 133,322 Pa). Dieser Unterdruckbereich bis höchstens 250 mm Hg hat sich als für die Wundheilung geeignet erwiesen. Ein bevorzugter Unterdruckbereich liegt zwischen 10 und 150 mm Hg.

Der unter Verwendung der Vorrichtung an die Wunde angelegte Unterdruck kann bei einer typischen Unterdruckbehandlung entweder zeitlich im Wesentlichen konstant gehalten werden, oder er kann zeitlich verändert, insbesondere zyklisch verändert werden, was über eine entsprechend ausgebildete und programmierte Steuervorrichtung bei der Unterdruck erzeugenden Einrichtung, insbesondere in Abhängigkeit weiterer Parameter, realisiert werden kann.

Zum Anlegen von Unterdruck und vorzugsweise auch zum Absaugen von Körperflüssigkeiten ist eine vorzugsweise flexible Saugleitung, beispielsweise in Form eines Drainageschlauchs, vorgesehen, der einenends über einen sogenannten Port im Bereich des Wundabdeckmaterials mit der Wundumgebung oder dem Wundraum und anderenends mit dem eingangs erwähnten Behälter zur Aufnahme von Körperflüssigkeiten bzw. mit der Unterdruck erzeugenden Einrichtung kommuniziert.

Der erste und der zweite Gehäuseteil sind, insbesondere zum Austausch eines vollen gegen einen unbenutzten Behälter, welcher durch den zweiten Gehäuseteil gebildet wird, manuell lösbar aneinander fixierbar, wobei im befestigten Zustand das Innere des Behälters über geeignete Anschlussmittel von der Unterdruck erzeugenden Einrichtung mit Unterdruck beaufschlagbar ist, so dass eine Unterdruckkommunikation zwischen der Unterdruck erzeugenden Einrichtung, dem Behälter und der zum Körper führenden Saugleitung herstellbar ist.

DE 10 2010 060 543 A1 offenbart eine Vorrichtung mit den Oberbegriffsmerkmalen des Anspruchs 1 und schlägt vor, den Behälter zur Aufnahme von aus einer Wunde abgeführten Flüssigkeiten von innen mit einer SAP-haltigen Einlage auszukleiden.

Aus wirtschaftlichen Gründen, Gewichtsbetrachtungen und aus herstellungstechnischen Gründen und zur Erzielung einer großen Aufnahmekapazität erweist es sich als vorteilhaft, wenn der Behälter weitestgehend hohl, also vorzugsweise frei von massiven Volumenbereichen ausgebildet ist; dies spart Gewicht und Material und eignet sich für eine Spritzgussfertigung in Zweischalentechnik. Da der Behälter bestimmungsgemäß zur Aufnahme von Körperflüssigkeiten dient, stellt sich das Problem, dass beim Neigen der Vorrichtung, insbesondere im mobilen Betrieb, die in seinem Inneren aufgenommene Körperflüssigkeit dazu neigt, der Schwerkraft folgend zu fließen. Dies führt zum einen dazu, dass die Flüssigkeit in dem Behälter herumschwappt, was bei durchsichtig oder zumindest nicht völlig undurchsichtigen Materialien zu unangenehmen Vorstellungen führt. Zum anderen kann die Flüssigkeit so auch an im Behälter angeordnete Filter und an in den Behälter mündende Anschlüsse gelangen, daran anhaften und diese zusetzen.

Der vorliegenden Erfindung liegt die Aufgabe zugrunde, den vorstehend geschilderten Problemen zu begegnen.

Diese Aufgabe wird durch eine Vorrichtung mit den Merkmalen des Anspruchs 1 gelöst.

Wenn vorstehend von einem SAP-haltigen, biegsamen Flachmaterialabschnitt die Rede ist, umfasst dies im Sinne der vorliegenden Erfindung einen Flachmaterialabschnitt, der zumindest teilweise ein superabsorbierendes Polymermaterial umfasst. Superabsorbierende Polymere (kurz: SAP) sind Stoffe, die in der Lage sind, ein Vielfaches ihres Eigengewichts an Flüssigkeiten aufzusaugen. Bei der Aufnahme der Flüssigkeit quillt der Superabsorber auf und bildet eine hydrogelierte Masse. In den Behälter eingesogene Körperflüssigkeit wird also von dem SAP-haltigen Flachmaterialabschnitt aufgenommen, zu einer gelartigen Masse verdickt und fließt somit nicht frei in dem Behälter herum. Je mehr Flüssigkeit in den Behälter eingesogen wird, desto größer wird das Volumen der hydrogelierten Masse im Inneren des Behälters. Ist das Innere des Behälters vollständig von dieser Masse ausgefüllt, kann der Behälter von der Vorrichtung gelöst und ausgetauscht werden.

Der SAP-haltige Flachmaterialabschnitt ist biegsam und somit flexibel an einen Wandungsverlauf des Behälters anpassbar, wobei der Flachmaterialabschnitt derart in den Behälter eingelegt ist, dass er dem Verlauf einer Wandung zumindest bereichsweise folgt. Der Flachmaterialabschnitt berührt dabei nicht zwingend die Wandung des Behälters, sondern verläuft abschnittsweise entlang dem Verlauf dieser Wandung und insbesondere parallel hierzu. Der Flachmaterialabschnitt ist also auch nicht zentral im Inneren des Behälters angeordnet, sondern nahe an der Wandung des Behälters positioniert und ist in dieser Position lagestabilisiert. Im Sinne der vorliegenden Erfindung bedeutet dies, dass der Flachmaterialabschnitt in der in den Behälter eingelegten Position stabilisiert ist und nicht lose im Behälter angeordnet ist oder beim Bewegen des Behälters oder der Vorrichtung gar lose darin umher fallen kann. Beispielsweise beim Neigen der Vorrichtung, insbesondere im mobilen Betrieb oder beim Fixieren oder Lösen des Behälters, wird auf diese Weise erreicht, dass der Flachmaterialabschnitt nicht der Schwerkraft folgend aus dieser Position bewegt wird und/oder in sich zusammenfällt.

In weiterer Ausbildung der Erfindung erweist es sich als vorteilhaft, wenn der Flachmaterialabschnitt einen zentral im Inneren des Behälters ausgebildeten flachmaterialabschnittfreien Raum oder Freiraum zumindest abschnittsweise umgibt. Der Flachmaterialabschnitt ist also nicht zentral im Inneren des Behälters angeordnet, sondern begrenzt den zentral im Inneren ausgebildeten flachmaterialabschnittfreien Raum oder Freiraum zumindest abschnittsweise. Quillt der Flachmaterialabschnitt bei der Aufnahme von Flüssigkeit auf, kann sich der Flachmaterialabschnitt zumindest teilweise in den im Inneren ausgebildeten Raum hinein ausdehnen.

In weiterer Ausbildung der Erfindung erweist es sich als vorteilhaft, wenn der Anschluss über wenigstens eine in einer Innenseite des Behälters mündende Einlassöffnung mit dem Inneren des Behälters kommuniziert, um Körperflüssigkeiten in den Flachmaterialabschnitt einzuleiten. Nach diesem weiteren Erfindungsgedanken ragt oder mündet die Einlassöffnung nicht mittig in das Innere des Behälters hinein, sondern mündet in der Innenseite, insbesondere in einer Innenseite der Wandung des Behälters. Auf diese Weise kann die Körperflüssigkeit von außen in den Flachmaterialabschnitt eingeleitet werden.

Es erweist sich als besonders vorteilhaft, wenn der Anschluss über mehrere Einlassöffnungen in das Innere des Behälters mündet. Auf diese Weise kann die Kommunikation zwischen dem Anschluss und dem Inneren des Behälters auch dann gewährleistet werden, falls eine der Einlassöffnungen verstopft oder zugesetzt ist.

In weiterer Ausbildung der Erfindung erweist es sich als vorteilhaft, wenn der SAP-haltige, biegsame Flachmaterialabschnitt an einer oder mehreren der Einlassöffnungen anliegt oder zumindest wenigstens nahezu daran angrenzt. Auf diese Weise wird sichergestellt, dass die Körperflüssigkeit unmittelbar beim Eintreten in den Behälter auf den Flachmaterialabschnitt trifft und von diesem aufgesaugt wird. Die Körperflüssigkeit verteilt sich also nicht erst in dem Behälter sondern wird direkt beim Eindringen von dem Flachmaterialabschnitt absorbiert.

In diesem Zusammenhang erweist es sich als vorteilhaft, wenn Körperflüssigkeiten von außen und quer zur flächenhaften Erstreckung des Flachmaterialabschnitts in den Flachmaterialabschnitt einleitbar sind.

Es erweist sich als besonders vorteilhaft, wenn im Inneren des Behälters wenigstens ein weiterer Flachmaterialabschnitt angeordnet ist. Mit mehreren separat voneinander angeordneten Flachmaterialabschnitten kann ein möglichst großer Bereich im Inneren des Behälters von den Flachmaterialabschnitten bedeckt werden. Es ist ebenfalls denkbar, wenigstens zwei Flachmaterialabschnitte zumindest teilweisen überschneidend oder überlappend anzuordnen, um die Absorptionskapazität bereichsweise zu erhöhen.

In weiterer Ausbildung der Erfindung erweist es sich als vorteilhaft, wenn wenigstens ein Flachmaterialabschnitt entlang dem ersten Wandungsteil angeordnet und wenigstens ein weiterer Flachmaterialabschnitt entlang dem zweiten Wandungsteil angeordnet ist. Vorteilhafterweise wird der jeweilige Flachmaterialabschnitt vor dem Fügen der beiden Wandungsteile gegeneinander in das jeweilige Wandungsteil eingelegt.

Erfindungsgemäß wird vorgeschlagen, dass der wenigstens eine Flachmaterialabschnitt mehreren, im Inneren des Behälters angeordneten Stiften oder Streben lagestabilisiert ist. Es ist denkbar, dass der Flachmaterialabschnitt zwischen einem Stift oder einer Strebe und einer Wandung des Behälters gehalten wird. Ebenfalls ist es denkbar, dass das Material von mehreren, insbesondere verteilt im Inneren des Behälters angeordneten Stiften bzw. Streben gehalten wird.

Weiter erfindungsgemäß ist der Stift oder die Strebe an dem ersten oder an dem zweiten Wandungsteil angeordnet und erstreckt sich ausgehend von dem ersten oder dem zweiten Wandungsteil in das Innere des Behälters. Vorteilhafterweise sind die Stifte oder Streben einstückig mit dem jeweiligen Wandungsteil verbunden und in einem Spritzgussverfahren hergestellt. Für den Herstellungsprozess erweist es sich als vorteilhaft, wenn sich der Stift oder die Strebe aus ausgehend von dem ersten oder zweiten Wandungsteil in einer Entformungsrichtung des jeweiligen Wandungsteils erstreckt.

Des Weiteren erweist es sich als vorteilhaft und zweckmäßig, wenn sich der Stift oder die Strebe in einer Fügerichtung des ersten und/oder zweiten Wandungsteils, insbesondere bis nahezu an das andere Wandungsteil angrenzend, erstreckt. Vorteilhafterweise erstreckt sich der Stift oder die Strebe ausgehend von dem einen Wandungsteil insbesondere bis zu 5mm, vorzugsweise bis zu 3mm, an das andere Wandungsteil heran. Ist der Flachmaterialabschnitt beispielsweise in den zweiten Wandungsteil eingelegt, kann dieser von einer oder mehreren ausgehend vom ersten Wandungsteil in das Innere des Behälters sich erstreckenden Stifte oder Streben, die sich nahezu bis an das zweite Wandungsteil heran erstrecken, lagestabilisiert werden.

Nach einem weiteren Erfindungsgedanken erweist es sich als vorteilhaft, wenn ein Flachmaterialabschnitt mehrere in einer Fläche nebeneinander angeordnete Flachmaterialabschnitt-teilstücke umfasst. Das Flachmaterial wird beispielsweise in viereckige Flachmaterialabschnittteilstücke, insbesondere in Flachmaterialabschnittteilstücke einer Standardgröße, geschnitten, und anschließend nebeneinander angeordnet, sodass sich ein Flachmaterialabschnitt in einer gewünschten Form ergibt. Auf diese Weise kann das passgenaue Zuschneiden des Flachmaterials und damit verbundene Kosten beim Fertigungsprozess vermieden werden. Beispielsweise kann Schnittabfall bei der Herstellung L-förmiger Flächen vermieden werden.

Es erweist sich weiter als zweckmäßig und vorteilhaft, wenn ein Flachmaterialabschnitt einen Zellstoffanteil und einen SAP-Anteil umfasst, wobei der Zellstoffanteil 35% - 45% beträgt und der SAP-Anteil 55% - 65% beträgt. Das Mischverhältnis der beiden Bestandteile ist zweckmäßig und vorteilhafterweise derart aufeinander abgestimmt, dass die in den Behälter eingesogene Flüssigkeit sich nicht zu schnell in dem Flachmaterialabschnitt ausbreitet aber auch nicht zu langsam von diesem aufgenommen wird. Ist der Zellstoffanteil zu hoch und der SAP-Anteil zu niedrig, kann die Flüssigkeit nicht gebunden werden. Ist im umgekehrten Fall der Zellstoffanteil zu niedrig und der SAP-Anteil zu hoch, wird die Flüssigkeit zu langsam aufgenommen.

Des Weiteren erweist es sich als vorteilhaft, wenn ein Flachmaterialabschnitt eine umhüllte SAP-Einlage umfasst. Eine umhüllte SAP-Einlage umfasst einen Kern aus einem superabsorbierenden Material und eine flüssigkeitsdurchlässige Umhüllung. Die Umhüllung ermöglicht eine staubarme Handhabbarkeit des superabsorbierenden Materials und verbessert auf dieses Weise die Handhabbarkeit des Flachmaterialabschnitts beim Einlegen in den Behälter bzw. in die Wandungsteile des Behälters.

Nach einer vorteilhaften Weiterbildung der Erfindung, weist der Behälter einen Bereich zur Aufnahme eines Filters auf. Mit Hilfe des Filters soll das Eindringen von Flüssigkeit in die Unterdruck erzeugende Einrichtung verhindert werden. Zweckmäßigerweise ist der Bereich zur Aufnahme des Filters im Bereich der Anschlussmittel angeordnet, über welche das Innere des Behälters von der Unterdruck erzeugenden Einrichtung mit Unterdruck beaufschlagbar ist.

Es erweist sich als zweckmäßig und vorteilhaft, wenn der Bereich zur Aufnahme des Filters zumindest teilweise durch Rippen, Stifte oder Stege, insbesondere käfigartig, von einem Bereich zur Aufnahme des Flachmaterialabschnitts im Inneren des Behälters abgegrenzt oder partitioniert ist. Vorteilhafterweise erstrecken sich die Rippen, Stifte oder Stege ausgehend von dem ersten und/oder zweiten Wandungsteil bis zumindest nahezu an das andere Wandungsteil heran und begrenzen somit einen Bereich zur Aufnahme des Filters. Ganz besonders bevorzugt erstrecken sich Rippen, Stifte oder Stege ausgehend von dem ersten und an entsprechenden Stellen ausgehend von dem zweiten Wandungsteil, so dass sich die Rippen, Stifte oder Stege beim Verbinden der beiden Wandungsteile komplementär ergänzen und so den Bereich zur Aufnahme des Filters käfigartig begrenzen. Dieser Bereich ist zweckmäßig von einem Bereich zur Aufnahme des Flachmaterialabschnitts im Inneren des Behälters abgegrenzt oder partitioniert. Mit Hilfe der Rippen, Stifte oder Stege kann verhindert werden, dass der Flachmaterialabschnitt in den Bereich zur Aufnahme des Filters hineinragen und mit einem darin angeordneten Filter in direkten körperlichen Kontakt kommen und diesen berühren kann. Vorzugsweise wird die gesamte in den Behälter eingesogene Körperflüssigkeit von dem Flachmaterialabschnitt aufgenommen, sodass wirksam verhindert werden kann, dass Körperflüssigkeit auf den Filter gelangt und diesen zusetzt.

Es erweist sich als besonders vorteilhaft, wenn eine Einlassöffnung in einem Bereich des Behälters gegenüber, vorzugsweise diagonal gegenüber, dem im Behälter angeordneten Filter mündet. Es ist denkbar, dass die Einlassöffnung an einer beliebigen Stelle in das Innere des Behälters mündet. Vorzugsweise ist die Einlassöffnung aber derart angeordnet, dass sie in einem vom Filter beabstandeten Bereich, vorzugsweise in einem Bereich des Behälters gegenüber, vorzugsweise diagonal gegenüber dem im Behälter angeordneten Filter mündet. Auf diese Weise kann verhindert werden, dass Körperflüssigkeit in den Bereich des Filters gelangen kann ohne von dem Flachmaterialabschnitt aufgesaugt zu werden.

Der Filter selbst kann beispielsweise block- oder scheibenförmig ausgebildet sein. Indessen erweist es sich als vorteilhaft, wenn der Filter zylindrisch, topf- oder konus- oder kegelmantelförmig ausgebildet ist, da hierdurch eine größere Durchdringungsfläche des Filters zur Verfügung steht. Ungeachtet der konkreten Gestalt des Filters ist er vorteilhafterweise aus einem starren und formstabilen Material ausgebildet. Als Filtermaterial geeignet sind beispielsweise poröse Feststoffe aus Kunststoff (beispielsweise aus Polyethylen oder Polypropylen) oder aus Keramik oder Metall.

Weiterhin kann eine Beimischung einer quellbaren Substanz (beispielsweise Carboxymethylzellulose) vorhanden sein, welche nach Kontakt mit Flüssigkeit die im Filtermaterial vorhandenen Poren verschließt und so einen weiteren Flüssigkeitsdurchtritt durch den Filter verhindert. Vorteilhaft ist auch die Beimischung einer geruchsabsorbierenden Substanz (beispielsweise Aktivkohle), welche gasförmige Stoffe, die eine Geruchsbelästigung verursachen können, zurückhält. Die geruchsabsorbierende Substanz kann auch als Beschichtung vorhanden sein.

Nach einem weiteren Erfindungsgedanken erweist es sich als vorteilhaft, wenn der Anschluss über einen entlang des Verlaufs einer Wandung erstreckten Kanal mit der Einlassöffnung kommuniziert. Die durch die Saugleitung abgesaugte Körperflüssigkeit wird über den Anschluss in den Kanal und über den Kanal zur Einlassöffnung geleitet. Auf diese Weise kann der Anschluss für die zum Körper führende Saugleitung ortsunabhängig von der Einlassöffnung am Behälter angeordnet werden, da der Anschluss über den Kanal mit der Einlassöffnung verbunden ist.

Es erweist sich als vorteilhaft, wenn der Kanal von dem ersten und zweiten Wandungsteil begrenzt wird. Vorteilhafterweise wird der Kanal beim Zusammenfügen der beiden Wandungsteile zwischen diesen begrenzt und ausgebildet, sodass der Kanal sowohl von dem ersten als auch von dem zweiten Wandungsteil begrenzt wird.

Es erweist sich als zweckmäßig und vorteilhaft, wenn der Kanal mehrere Einlassöffnungen ins Innere des Behälters bildet oder aufweist. Auf diese Weise kann die Kommunikation zwischen dem Anschluss und dem Inneren des Behälters gewährleistet werden, falls eine Einlassöffnung verstopft oder zugesetzt sein sollte.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform der erfindungsgemäßen Vorrichtung. Für die Merkmale der Patentansprüche wird jeweils separat und losgelöst von einer Rückbeziehung der Ansprüche sowie in beliebiger Kombination Schutz in Anspruch genommen. In der Zeichnung zeigt:
- Fig. 1a und 1b: verschiedene Ansichten einer bevorzugten Ausführungsform der erfindungsgemäßen Vorrichtung zur Bereitstellung von Unterdruck für medizinische Anwendungen;
- Fig. 2a: den ersten und zweiten Gehäuseteil der Vorrichtung in einem zusammengefügten Zustand;
- Fig. 2b: den ersten und zweiten Gehäuseteil der Vorrichtung in einem voneinander gelösten Zustand;
- Fig. 3a bis 3f: verschiedene Ansichten des den zweiten Gehäuseteil der Vorrichtung bildenden Behälters, und
- Fig. 4a bis 4c: verschiedene Ansichten der den Behälter bildenden Wandungsteile.

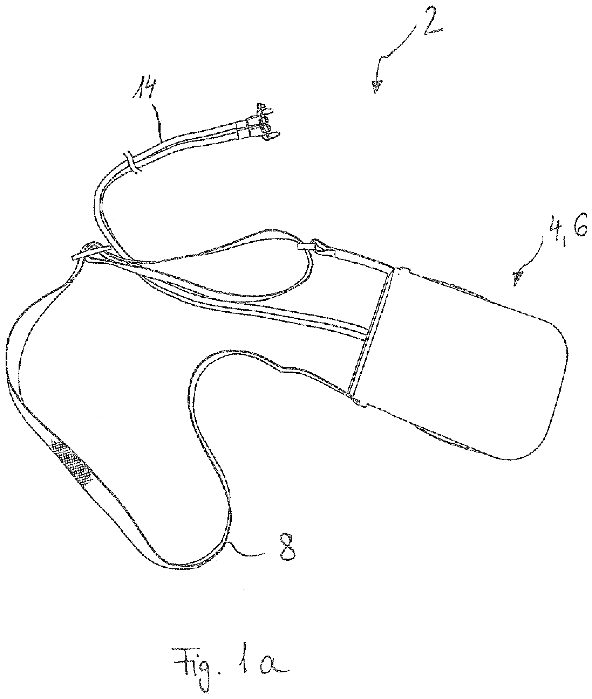

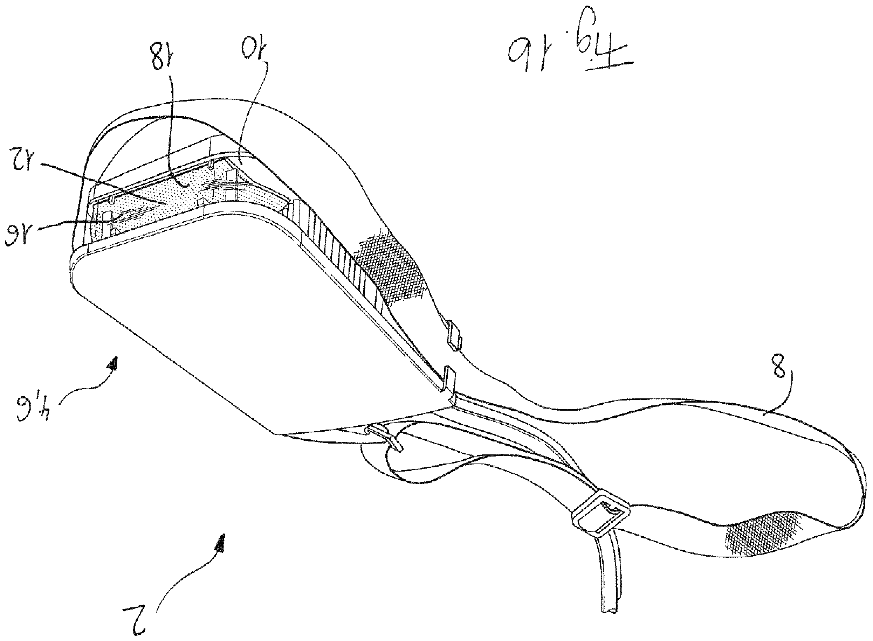

Die Figuren 1a und 1b zeigen eine erste Ausführungsform einer erfindungsgemäßen tragbaren Vorrichtung 2 zur Bereitstellung von Unterdruck für medizinische Anwendungen. Die Vorrichtung 2 umfasst zwei lösbar gegeneinander fixierbare Gehäuseteile 4, 6, sowie ein gurtartiges Haltemittel 8, so dass die Vorrichtung 2 am Körper eines Benutzers tragbar und mitführbar ist. Das Haltemittel 8 ist vorzugsweise ein flexibles streifenförmiges, riemenartiges längenverstellbares Haltemittel 8, insbesondere aus Nylongewebe oder einem beliebigen anderen Material. In dem ersten Gehäuseteil 4 ist eine Unterdruck erzeugende Einrichtung vorzugsweise in Form einer Luftpumpe sowie elektrische und elektronische Steuerkomponenten einschließlich Batterien oder vorzugsweise wiederaufladbarer Akkus aufgenommen. Der zweite Gehäuseteil 6 bildet im bevorzugten Fall zugleich einen Behälter 10 zur Aufnahme von Körperflüssigkeiten, insbesondere zur Aufnahme von aus einer Wunde abgesaugten Wundsekreten. Im zusammen gefügten Zustand der beiden Gehäuseteile 4,6 ist ein Inneres 12 des Behälters 10 über geeignete Anschlussmittel von der Unterdruck erzeugenden Einrichtung mit Unterdruck beaufschlagbar. Von dem Behälter 10 führt eine Saugleitung 14 zum Körper eines Patienten. Es ist denkbar, dass der Behälter 10 bzw. das zweite Gehäuseteil 6 zumindest teilweise aus einem durchsichtigen Material gefertigt ist. Fig. 1b zeigt einen solchen durchsichtigen Bereich 16, über den eine Einsichtnahme in das Innere 12 des Behälters 10 möglich ist. Weiter ist in Fig. 1b ersichtlich, dass im Inneren 12 des Behälter 10 ein SAP-haltiger, biegsamer Flachmaterialabschnitt 18 angeordnet ist.

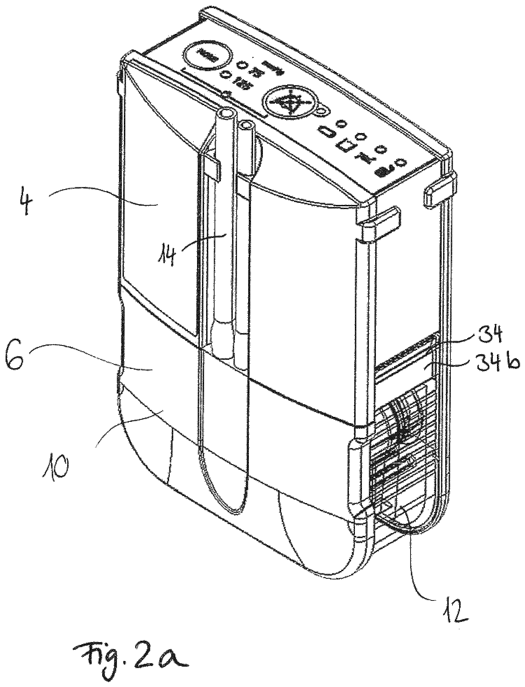

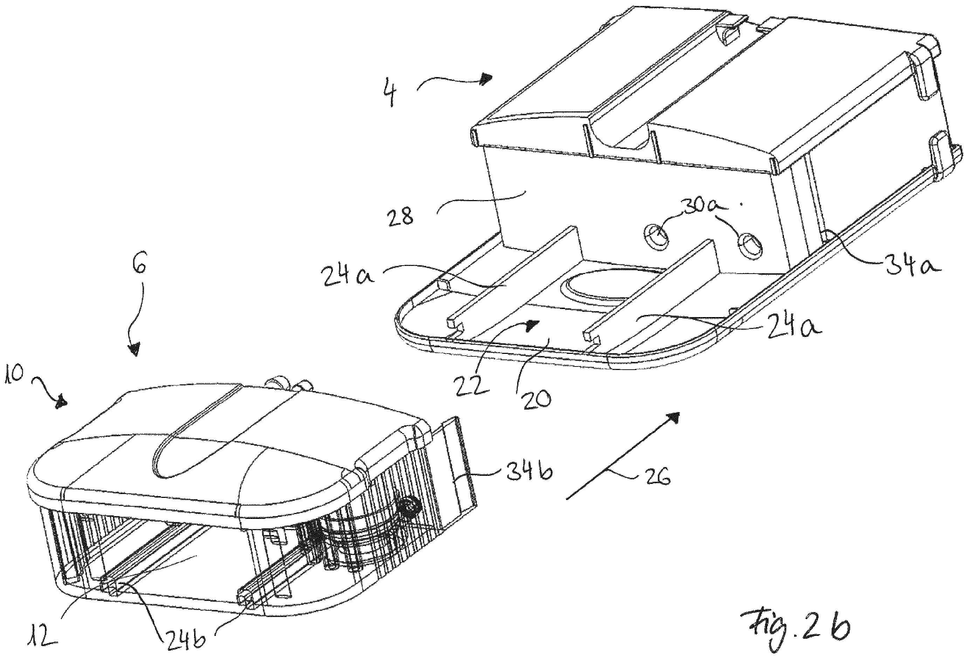

Die Figuren 2a und 2b zeigen den ersten und zweiten Gehäuseteil 4, 6 der Vorrichtung 2 in einem zusammengefügten bzw. in einem voneinander gelösten Zustand. Der in den Behälter 10 eingelegte Flachmaterialabschnitt 18 ist in den Fig. 2a und 2b nicht dargestellt. Man erkennt bei der dargestellten bevorzugten Ausführungsform in der Figur 2b, dass der erste Gehäuseteil 4 in der Seitenansicht ein L-förmiges Profil mit einer vorstehenden Schenkelplatte 20 aufweist. An einer Innenseite 22 der Schenkelplatte 20 des ersten Gehäuseteils 4 sind beispielhaft zwei erste Schiebeführungsmittel 24a ausgebildet. Der zweite Gehäuseteil 6 umfasst zu den ersten Schiebeführungsmitteln 24a komplementäre zweite Schiebeführungsmittel 24b. Hierdurch ist der zweite Gehäuseteil 6 auf die Schenkelplatte 20 des ersten Gehäuseteils 4 translatorisch geführt in einer Schieberichtung 26 aufschiebbar. Die ersten Schiebeführungsmittel 24a des ersten Gehäuseteils 4 sind in der gezeigten Ausführungsform als Führungsschienen oder - rippen ausgebildet. Die zweiten Schiebeführungsmittel 24b sind als dazu komplementäre Aufnahmevertiefungen, insbesondere in Form von Schiebeführungsnuten, ausgebildet. Wenn die ersten Schiebeführungsmittel 24a in Eingriff mit den zweiten Schiebeführungsmitteln 24b sind, lässt sich der zweite Gehäuseteil 6 translatorisch auf den ersten Gehäuseteil 4 aufschieben, bis die Gehäuseteile im bestimmungsgemäß zusammengesetzten Zustand (Fig. 2a) auf Stoß aneinander anliegen und der zweite Gehäuseteil 6 das erste Gehäuseteil 4 komplementär ergänzt.

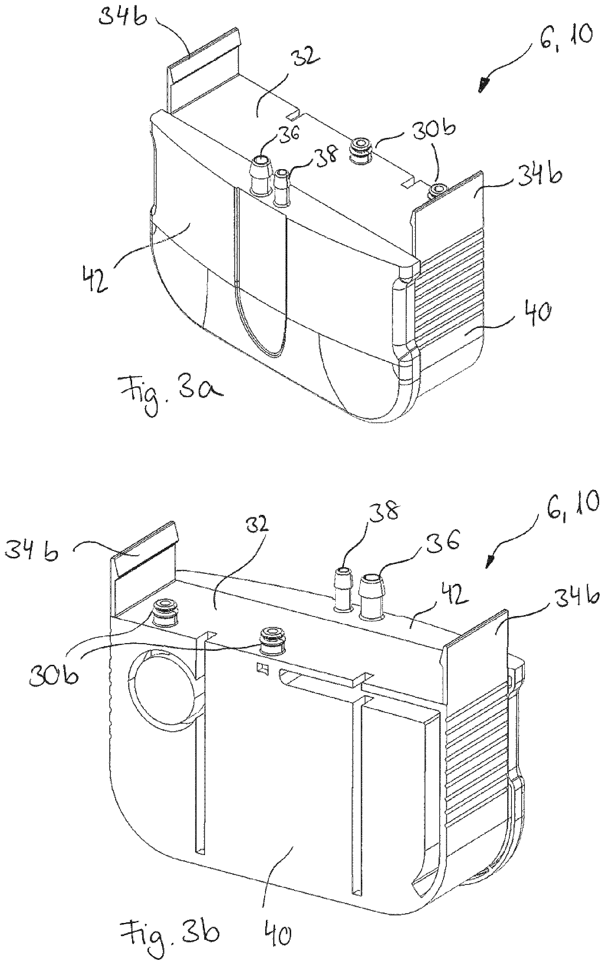

Aus der Figur 2b, welche die beiden Gehäuseteile 4,6 separat voneinander zeigt, ist unmittelbar ersichtlich, dass auf einer dem zweiten Gehäuseteil 6 zugewandten Seite 28 des ersten Gehäuseteils 4 mehrere Anschlussmittel 30a ausgebildet sind, die mit Anschlussmitteln 30b (vgl. Fig. 3a) des zweiten Gehäuseteils 6 koppelbar sind, wenn die Gehäuseteile 4,6 miteinander gefügt werden. Die dem ersten Gehäuseteil 4 zugewandte Seite 32 des zweiten Gehäuseteils 6 ist komplementär zu der Seite 28 des ersten Gehäuseteils 4 ausgebildet, so dass die beiden Gehäuseteile 4, 6 nur in einer korrekten Weise miteinander gefügt bzw. aneinander befestigt werden können.

Des Weiteren umfasst die Vorrichtung 2 schnappende, rastende oder in sonstiger Weise hintergreifend wirkende Verriegelungsmittel 34, mittels derer der zweite Gehäuseteil 6 gegen den ersten Gehäuseteil 4 lösbar fixierbar ist. Durch translatorisches Aufschieben des zweiten Gehäuseteils 6 auf den ersten Gehäuseteil 4 werden am zweiten Gehäuseteil 6 angeordnete Verriegelungslaschen 34b, insbesondere selbsttätig, ausgelenkt und rasten dann in eine die Gehäuseteile 4, 6 gegeneinander verriegelnde Stellung. Hierfür sind an dem ersten Gehäuseteil 4 zu den Verriegelungslaschen 34b komplementäre hintergreifbare Elemente 34a vorgesehen, die von den Verriegelungslaschen 34b hintergriffen werden. Wenn die Gehäuseteile 4, 6 zusammengefügt sind, wird vorzugsweise automatisch eine Unterdruckkommunikation zwischen dem Inneren des Behälters 10 des zweiten Gehäuseteils 6 und der Unterdruck erzeugenden Einrichtung hergestellt. Vorzugsweise sind die Verriegelungsmittel 34, 34a und 34b selbsttätig lösbar, wenn der zweite Gehäuseteil 6 in einer translatorischen Zugbewegung in einer Richtung entgegen der Schieberichtung 26 von dem ersten Gehäuseteil 4 weggezogen wird.

Die Figuren 3a bis 3d zeigen verschiedene Ansichten und Ausführungsformen des das zweite Gehäuseteil 6 bildenden Behälters 10 ohne den eingelegten Flachmaterialabschnitt 18. Auf der Seite 32 des Behälters sind neben den Anschlussmitteln 30b weitere Anschlüsse 36 und 38 ausgebildet. Der Anschluss 36 ist für die Saugleitung 14 (vgl. Fig. 1a) vorgesehen, die dann beispielsweise bei der Verwendung der Vorrichtung 2 zur Unterdrucktherapie von Wunden zu einem die Wunde druckdicht verschließenden Wundverband führt und dort beispielsweise über einen Port mit dem Wundraum kommuniziert, um im Wundraum einen Unterdruck anzulegen und aufrechtzuerhalten und Wundsekrete in den Behälter 10 abzusaugen. Hierfür kommuniziert der Behälter 10 über die Anschlussmittel 30b mit der Unterdruck erzeugenden Einrichtung, die in dem ersten Gehäuseteil 4 angeordnet ist. Bei dem weiteren Anschlussmittel 38 kann es sich um einen Instillations- oder Spülanschluss oder um einen Druckmessanschluss handeln.

Der Behälter 10 umfasst ein erstes und ein zweites Wandungsteil 40 und 42. Die beiden Wandungsteile 40, 42 werden vorzugsweise durch Kleben oder Thermoschweißen unlösbar und dichtend gegeneinander gefügt und bilden so den Behälter 10. Es ist denkbar, dass die Wandungsteile 40, 42 halbschalenförmig ausgebildet sind und im zusammengefügten Zustand einen scheibenförmigen Behälter bilden. In den dargestellten Ausführungsformen ist das Wandungsteil 40 schachtelförmig mit einer offenen Seite, die durch das deckelartige Wandungsteil 42 verschlossen wird, ausgebildet.

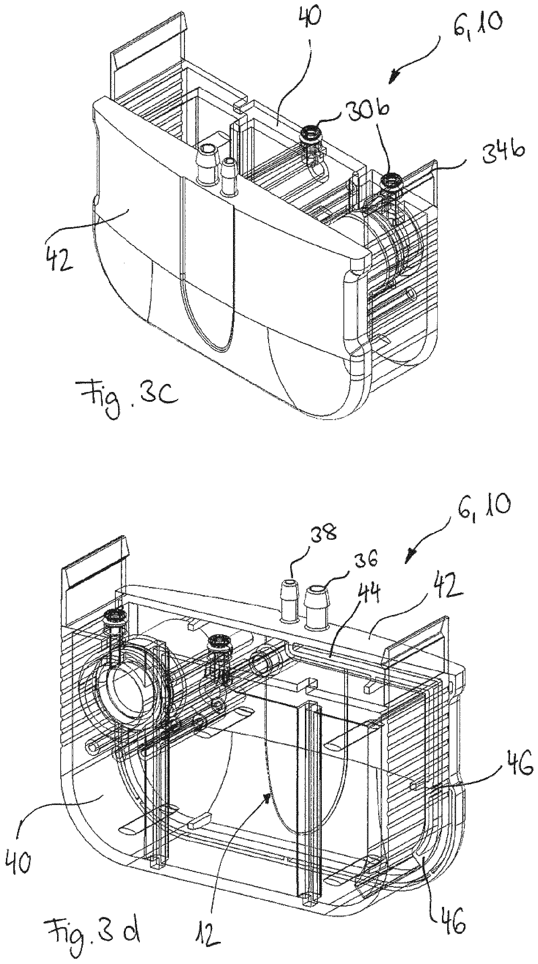

Zum besseren Verständnis wurde das erste Wandungsteil 40 des Behälters in den Figuren 3c und 3d durchsichtig oder zumindest teilweise durchsichtig dargestellt. An dieser Stelle sei erwähnt, dass es sich dabei um eine beispielhafte Darstellung handelt. Es ist denkbar, dass der zweite Gehäuseteil 6 oder der Behälter 10 an mehreren Seiten oder insbesondere ganz oder überhaupt nicht durchsichtig ausgebildet ist.

Aus Figur 3d ist ersichtlich, dass in dem Behälter 10 ein Kanal 44 ausgebildet ist. Dieser wird vorzugsweise von dem ersten und dem zweiten Wandungsteil 40, 42 begrenzt. Der Kanal 44 mündet über mehrere Einlassöffnungen 46 in das Innere 12 des Behälters 10, so dass die zum Körper führende Saugleitung 14 über den Anschluss 36 und den daran anschließenden Kanal 44 über die Einlassöffnungen 46 mit dem Inneren 12 des Behälters kommunizieren kann. Wird der Behälter 10 von der Unterdruck erzeugenden Einrichtung über die Anschlussmittel 30b mit Unterdruck beaufschlagt, wird über die Saugleitung 14 Flüssigkeit, insbesondere aus einer Wunde abgesaugte Körperflüssigkeit, in das Innere 12 des Behälters 10 eingesogen und tritt über die Einlassöffnungen 46 in das Innere des Behälters ein.

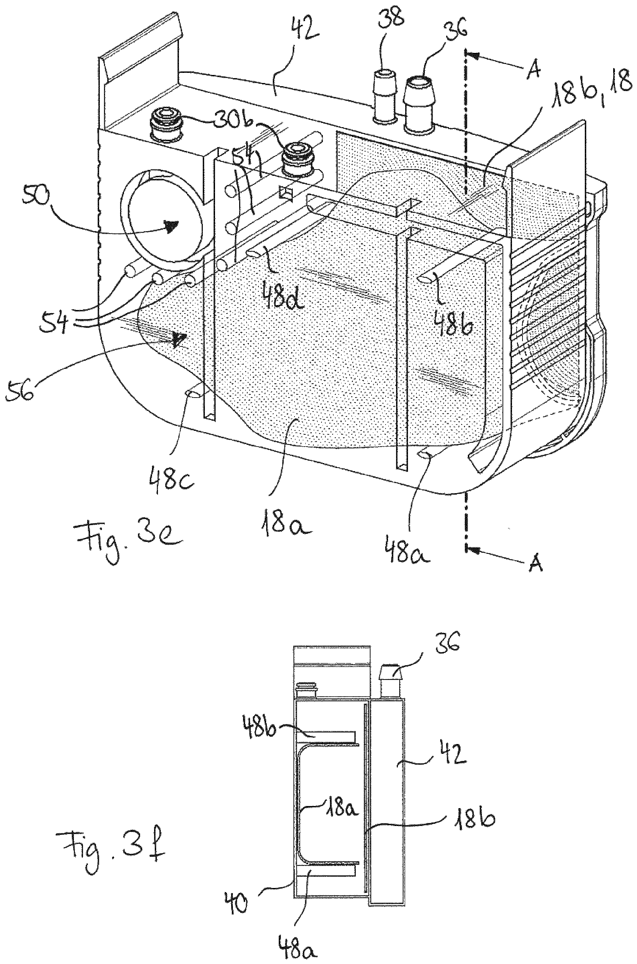

Die Figur 3e zeigt den Behälter 10 mit mehreren darin eingelegten biegsamen, SAP-halten Flachmaterialschnitten 18a und 18b. Ein erster Flachmaterialabschnitt 18a ist entlang dem Verlauf der Wandung des ersten Wandungsteils 40 in den Behälter eingelegt, und der zweite Flachmaterialabschnitt 18b ist entlang dem Verlauf der Wandung des zweiten Wandungsteils 42 in den Behälter eingelegt. Da die Flachmaterialabschnitte 18a, 18b flexibel biegsam sind, ist es möglich, den Flachmaterialabschnitt 18a entlang einem Verlauf einer Wandung des ersten Wandungsteils 40 in den Behälter einzulegen. Die Flachmaterialabschnitte 18a, 18b werden gemäß der dargestellten bevorzugten Ausführungsform durch mehrere sich in das Innere des Behälters hineinerstreckende Stifte der Streben 48a, 48b, 48c, 48d in der eingelegten Position lagestabilisiert.

Figur 3f ist eine skizzenhafte Schnittansicht des Behälters 10 gemäß der Schnittlinie A-A (vgl. Fig. 3e). Es ist ersichtlich, dass sich die Stifte oder Streben 48a, 48b ausgehend vom ersten Wandungsteil 40 des Behälters 10 in das Innere des Behälters bis nahe an das zweite Wandungsteil 42 heran erstrecken. Der erste Flachmaterialabschnitt 18a ist in der Schnittansicht bogenförmig in das erste Wandungsteil 40 eingelegt und wird von den Stiften oder Streben 48a, 48b in dieser Position lagestabilisiert. Der zweite Flachmaterialabschnitt 18b ist entlang dem Verlauf einer Wandung des zweiten Wandungsteils 42 an dieses angelegt und wird ebenfalls von den Stiften oder Streben 48a, 48b, die sich bis nahezu an das zweite Wandungsteil 42 heran erstrecken, in dieser Position lagestabilisiert.

Weiter ist aus der Figur 3e ersichtlich, dass ein Bereich 50 zur Aufnahme eines Filters 52 durch mehrere Rippen, Stifte oder Stege 54 von einem Bereich 56, in dem die Flachmaterialabschnitte im Inneren des Behälters eingelegt sind, abgegrenzt oder partitioniert ist. Mit Hilfe der Rippen, Stifte oder Stege 54 kann verhindert werden, dass die Flachmaterialabschnitte 18a, 18b in den Bereich 50 zur Aufnahme des Filters 52 hineinragen und mit einem darin angeordneten Filter 52 in direkten körperlichen Kontakt kommen.

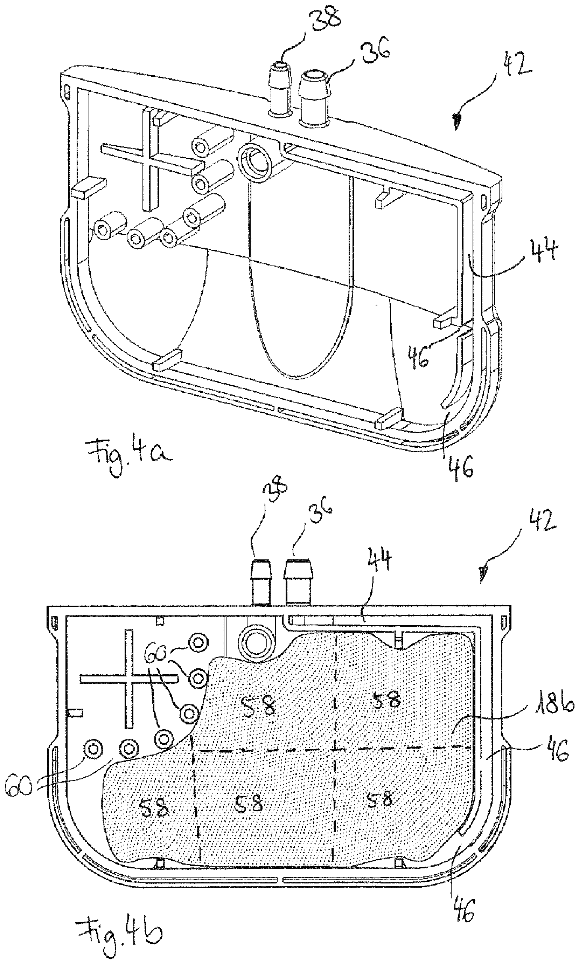

Die Figuren 4a und 4b zeigen das zweite Wandungsteil 42 mit eingelegtem Flachmaterialabschnitt 18b (Fig. 4b) und ohne eingelegten Flachmaterialabschnitt (Fig. 4a). Ausgehend von dem Anschluss 36 ist der Bereich des zweiten Wandungsteils zu erkennen, welcher durch Zusammenfügen mit dem ersten Wandungsteil den Kanal 40 bildet. Gemäß einer dargestellten bevorzugten Ausführungsform (vgl. Fig. 4b) liegt der Flachmaterialabschnitt an den Einlassöffnungen 46, über die der Kanal 44 in das Innere 12 des Behälters 10 mündet, an oder grenzt zumindest wenigstens nahezu daran an. Auf diese Weise wird sichergestellt, dass die Körperflüssigkeit unmittelbar beim Eindringen in den Behälter 10 auf den Flachmaterialabschnitt 18b trifft und von diesem aufgesaugt wird. Der Flachmaterialabschnitt 18b kann ein passgenauer Zuschnitt eines Flachmaterials sein. Der Flachmaterialabschnitt 18b kann auch aus mehreren Flachmaterialabschnittteilstücken 58 zu einem Flachmaterialabschnitt zusammengesetzt sein. Beispielsweise kann der Flachmaterialabschnitt 18b aus mehreren Flachmaterialabschnittteilstücken 58 gleicher oder unterschiedlicher Größe und/oder Form zusammengesetzt sein, wie es skizzenhaft in der Fig. 4b durch die gestrichelten Linien dargestellt ist.

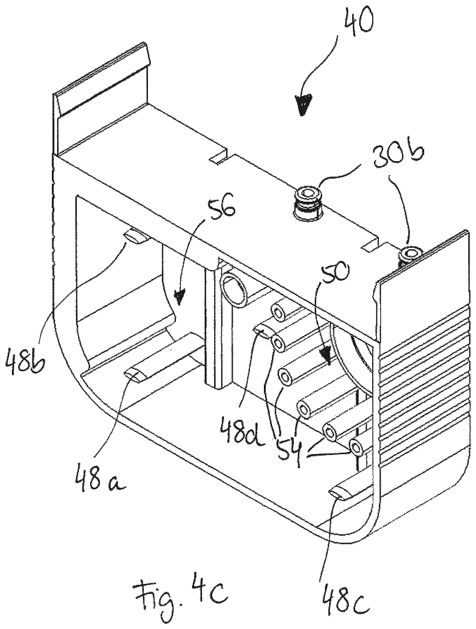

Weiter sind in den Figuren 4a und 4b mehrere vorstehende rohrförmige Ansätze 60 ersichtlich. Diese erstrecken sich ausgehend vom zweiten Wandungsteil 42 in eine Richtung, die der Fügerichtung des ersten und zweiten Wandungsteils 40, 42 entspricht. Das erste Wandungsteil 40 weist zu den rohrförmigen Ansätzen 60 komplementäre Rippen, Stifte oder Stege 54 auf, die sich ausgehend von dem ersten Wandungsteil 40 in Fügerichtung des ersten und zweiten Wandungsteils 40, 42 erstrecken (vgl. Fig. 4c). Beim Zusammensetzen der beiden Wandungsteile 40, 42 berühren sich die Rippen, Stifte oder Stege 54 und die rohrförmigen Ansätze 60 oder greifen ineinander ein und bilden auf diese Weise den Bereich 50 zur Aufnahme des Filters 52.

Weiter sind in Figur 4c die vier Stifte bzw. Streben 48a, 48b, 48c und 48d eingezeichnet, die sich ausgehend von dem ersten Wandungsteil 40 ebenfalls in Fügerichtung des ersten und zweiten Wandungsteils 40, 42 erstrecken. Sind die Wandungsteile 40, 42 zusammengesetzt, erstrecken sich die Stifte oder Streben 48a, 48b, 48c und 48d fast bis an des zweite Wandungsteil 42 heran und halten den in das zweite Wandungsteil 42 eingelegten Flachmaterialabschnitt 18b lagestabil.

## Patentansprüche

1. Vorrichtung (2) zur Bereitstellung von Unterdruck zur Unterdruckbehandlung von Wunden am menschlichen Körper, mit einer Unterdruck erzeugenden Einrichtung und einem nach Gebrauch wegwerfbaren Einweg-Behälter (10) zur Aufnahme von Flüssigkeiten, insbesondere von aus einer Wunde abgesaugten Wundsekreten, mit einem Anschluss (36) für eine zum Körper führende Saugleitung (14), wobei die Unterdruck erzeugende Einrichtung in einem ersten Gehäuseteil (4) der Vorrichtung (2) angeordnet ist und der Behälter (10) von einem zweiten Gehäuseteil (6) der Vorrichtung (2) gebildet ist, wobei der erste und der zweite Gehäuseteil (4, 6) lösbar gegeneinander fixierbar sind und wobei der Behälter (10) seinerseits wenigstens ein erstes und ein zweites Wandungsteil (40, 42) umfasst, welche unlösbar miteinander verbunden sind und dabei das Innere (12) des Behälters (10) begrenzen, wobei im Inneren (12) des Behälters (10) ein biegsamer SAP-haltiger Flachmaterialabschnitt (18) zur Aufnahme von in den Behälter (10) gesogenen Körperflüssigkeiten angeordnet ist, wobei der Flachmaterialabschnitt (18) dem Verlauf einer Wandung des Behälters (10) wenigstens bereichsweise folgend in den Behälter (10) eingelegt und in dieser Position lagestabilisiert ist, **dadurch gekennzeichnet, dass** der SAP-haltige Flachmaterialabschnitt (18) ^{,}von mehreren im Inneren des Behälters angeordneten Stiften oder Streben (48a, 48b, 48c, 48d) lagestabilisiert ist und dass der jeweilige Stift oder die Strebe (48a, 48b, 48c, 48d) an dem ersten oder an dem zweiten Wandungsteil (40, 42) angeordnet ist und sich ausgehend von dem ersten oder dem zweiten Wandungsteil (40, 42) in das Innere (12) des Behälters (10) erstreckt.

2. Vorrichtung (2) nach Anspruch 1, **dadurch gekennzeichnet, dass** der Flachmaterialabschnitt (18) einen zentral im Inneren des Behälters ausgebildeten flachmaterialabschnittfreien Raum oder Freiraum zumindest abschnittsweise umgibt.

3. Vorrichtung (2) nach Anspruch 1 oder 2, **dadurch gekennzeichnet, dass** der Anschluss (36) über wenigstens eine in einer Innenseite des Behälters (10) mündende Einlassöffnung (46) mit dem Inneren (12) des Behälters (10) kommuniziert, um Körperflüssigkeiten in den Flachmaterialabschnitt (18) einzuleiten.

4. Vorrichtung (2) nach Anspruch 3, **dadurch gekennzeichnet, dass** der Anschluss (36) über mehrere Einlassöffnungen (46) in das Innere (12) des Behälters (10) mündet.

5. Vorrichtung (2) nach Anspruch 3 oder 4, **dadurch gekennzeichnet, dass** der SAP-haltige, biegsame Flachmaterialabschnitt (18) an einer oder mehreren der Einlassöffnungen (46) anliegt oder zumindest wenigstens nahezu daran angrenzt.

6. Vorrichtung (2) nach Anspruch 5, **dadurch gekennzeichnet, dass** Körperflüssigkeiten von außen und quer zur flächenhaften Erstreckung des Flachmaterialabschnitts in den Flachmaterialabschnitt (18) einleitbar sind.

7. Vorrichtung (2) nach einem oder mehreren der vorhergehenden Ansprüche, **dadurch gekennzeichnet, dass** im Inneren (12) des Behälters (10) wenigstens ein weiterer Flachmaterialabschnitt (18a, 18b) angeordnet ist.

8. Vorrichtung (2) nach Anspruch 7, **dadurch gekennzeichnet, dass** wenigstens ein Flachmaterialabschnitt (18a) entlang dem ersten Wandungsteil (40) angeordnet und wenigstens ein weiterer Flachmaterialabschnitt (18b) entlang dem zweiten Wandungsteil (40) angeordnet ist.

9. Vorrichtung (2) nach einem oder mehreren der vorhergehenden Ansprüche, **dadurch gekennzeichnet, dass** sich der Stift oder die Strebe (48a, 48b, 48c, 48d) in einer Fügerichtung des ersten und/oder zweiten Wandungsteils (40, 42), insbesondere bis nahezu an das andere Wandungsteil (40, 42), erstreckt.

10. Vorrichtung (2) nach einem oder mehreren der vorhergehenden Ansprüche, **dadurch gekennzeichnet, dass** ein Flachmaterialabschnitt (18) mehrere in einer Fläche nebeneinanderangeordnete Flachmaterialabschnittteilstücke (58) umfasst.

11. Vorrichtung (2) nach einem oder mehreren der vorhergehenden Ansprüche, **dadurch gekennzeichnet, dass** der Behälter (10) einen Bereich (50) zur Aufnahme eines Filters (52) aufweist und
dass der Bereich (50) zur Aufnahme des Filters (52) zumindest teilweise durch Rippen, Stifte oder Stege (54), insbesondere käfigartig, von einem Bereich (56) zur Aufnahme des Flachmaterialabschnitts (18) im Inneren (12) des Behälters (10) abgegrenzt oder partitioniert ist.

12. Vorrichtung (2) nach Anspruch 11, **dadurch gekennzeichnet, dass** eine Einlassöffnung (46) in einem Bereich des Behälters (10) gegenüber, vorzugsweise diagonal gegenüber, dem im Behälter angeordneten Filter (52) mündet.

13. Vorrichtung (2) nach einem der Ansprüche 3 bis 12, **dadurch gekennzeichnet, dass** der Anschluss (36) über einen entlang des Verlaufs einer Wandung erstreckten Kanal (44) mit der Einlassöffnung (46) kommuniziert.

14. Vorrichtung (2) nach Anspruch 13, **dadurch gekennzeichnet, dass** der Kanal (44) von dem ersten und zweiten Wandungsteil (40, 42) begrenzt wird.

15. Vorrichtung (2) nach Anspruch 13 oder 14, **dadurch gekennzeichnet, dass** der Kanal (44) mehrere Einlassöffnungen (46) ins Innere (12) des Behälters (10) bildet oder aufweist.

## Claims

1. Device (2) for providing negative pressure for the negative pressure treatment of wounds on the human body, comprising an apparatus which generates negative pressure and a disposable container (10) which can be disposed of after use and is intended for receiving liquids, in particular wound secretions sucked out of a wound, comprising a connection (36) for a suction line (14) leading to the body, the apparatus that generates negative pressure being arranged in a first housing part (4) of the device (2) and the container (10) being formed by a second housing part (6) of the device (2), it being possible to detachably fix the first and the second housing part (4, 6) to one another and the container (10) in turn comprising at least a first and a second wall part (40, 42) which are non-detachably connected to one another and thus delimit the interior (12) of the container (10), a flexible, SAP-containing flat material portion (18) for receiving bodily fluids sucked into the container (10) being arranged in the interior (12) of the container (10), the flat material portion (18) being placed into the container (10) so as to follow the course of a wall of the container (10) at least in regions and being stabilized in this position, **characterized in that** the SAP-containing flat material portion (18) is stabilized in position by a plurality of pins or struts (48a, 48b, 48c, 48d) arranged in the interior of the container and **in that** the particular pin or strut (48a, 48b, 48c, 48d) is arranged on the first or on the second wall part (40, 42) and extends from the first or the second wall part (40, 42) into the interior (12) of the container (10).

2. Device (2) according to claim 1, **characterized in that** the flat material portion (18) at least partially surrounds a space or free space which is formed centrally in the interior of the container and is free of a flat material portion.

3. Device (2) according to claim 1 or claim 2, **characterized in that** the connection (36) communicates with the interior (12) of the container (10) via at least one inlet opening (46) that opens out in an inner face of the container (10), in order to introduce bodily fluids into the flat material portion (18).

4. Device (2) according to claim 3, **characterized in that** the connection (36) opens into the interior (12) of the container (10) via a plurality of inlet openings (46).

5. Device (2) according to claim 3 or claim 4, **characterized in that** the SAP-containing, flexible flat material portion (18) rests against one or more of the inlet openings (46) or at least almost abuts against them.

6. Device (2) according to claim 5, **characterized in that** bodily fluids can be introduced into the flat material portion (18) from the outside and transversely to the planar extension of the flat material portion.

7. Device (2) according to one or more of the preceding claims, **characterized in that** at least one further flat material portion (18a, 18b) is arranged in the interior (12) of the container (10).

8. Device (2) according to claim 7, **characterized in that** at least one flat material portion (18a) is arranged along the first wall part (40) and at least one further flat material portion (18b) is arranged along the second wall part (40).

9. Device (2) according to one or more of the preceding claims, **characterized in that** the pin or the strut (48a, 48b, 48c, 48d) extends in a joining direction of the first and/or second wall part (40, 42), in particular almost up to the other wall part (40, 42).

10. Device (2) according to one or more of the preceding claims, **characterized in that** a flat material portion (18) comprises a plurality of flat material portion pieces (58) arranged one next to the other in one surface.

11. Device (2) according to one or more of the preceding claims, **characterized in that** the container (10) has a region (50) for receiving a filter (52) and
**in that** the region (50) for receiving the filter (52) is at least partially delimited or partitioned by ribs, pins or projections (54), in particular in the manner of a cage, from a region (56) for receiving the flat material portion (18) in the interior (12) of the container (10).

12. Device (2) according to claim 11, **characterized in that** an inlet opening (46) opens out in a region of the container (10) opposite, preferably diagonally opposite, the filter (52) arranged in the container.

13. Device (2) according to any of claims 3 to 12, **characterized in that** the connection (36) communicates with the inlet opening (46) via a channel (44) extending along the course of a wall.

14. Device (2) according to claim 13, **characterized in that** the channel (44) is delimited by the first and the second wall part (40, 42).

15. Device (2) according to claim 13 or claim 14, **characterized in that** the channel (44) forms or comprises a plurality of inlet openings (46) into the interior (12) of the container (10).

## Revendications

1. Dispositif (2) destiné à fournir de la pression négative pour le traitement par pression négative de plaies sur le corps humain, avec un dispositif générateur de pression négative et un récipient jetable (10) qui peut être jeté après l'usage et qui est destiné à recevoir des liquides, en particulier des sécrétions de plaie aspirées au niveau d'une plaie, avec un raccord (16) pour une conduite d'aspiration (22) menant vers le corps, dans lequel le dispositif générateur de pression négative est agencé dans une première partie de boîtier (4) du dispositif (2) et le récipient (10) est formé par une deuxième partie de boîtier (6) du dispositif (2), dans lequel les première et deuxième parties de boîtier (4, 6) peuvent être fixées de manière amovible l'une à l'autre et dans lequel le récipient (10) comprend à son tour au moins une première et une deuxième partie de paroi (40, 42) qui sont reliées de manière inamovible l'une à l'autre tout en délimitant ainsi le volume intérieur (12) du récipient (10), dans lequel dans le volume intérieur (12) du récipient (10) est agencée une section de matériau plat (18) flexible contenant du SAP qui est destinée à recevoir des fluides corporels aspirés dans le récipient (10),dans lequel ladite section de matériau plat (18) est insérée dans le récipient (10) en suivant, au moins par zones, le tracé d'une paroi du récipient (10) et est stabilisée en position dans cette position, **caractérisé par le fait que** ladite section de matériau plat (18) contenant du SAP est stabilisée en position par une pluralité de broches ou d'entretoises (48a, 48b, 48c, 48d) qui sont agencées à l'intérieur du récipient et que la broche ou l'entretoise (48a, 48b, 48c, 48d) respective est agencée sur la première ou sur la deuxième partie de paroi (40, 42) et s'étend à partir de la première ou de la deuxième partie de paroi (40, 42) dans le volume intérieur (12) du récipient (10) .

2. Dispositif (2) selon la revendication 1, **caractérisé par le fait que** ladite section de matériau plat (18) entoure, au moins par sections, un espace exempt de section de matériau plat ou espace libre qui est formé de manière centrale à l'intérieur du récipient.

3. Dispositif (2) selon la revendication 1 ou 2, **caractérisé par le fait que** le raccord (36) communique avec le volume intérieur (12) du récipient (10) par au moins une ouverture d'entrée (46) débouchant dans une face intérieure du récipient (10), pour introduire des fluides corporels dans la section de matériau plat (18) .

4. Dispositif (2) selon la revendication 3, **caractérisé par le fait que** le raccord (36) débouche dans le volume intérieur (12) du récipient (10) par une pluralité d'ouvertures d'entrée (46).

5. Dispositif (2) selon la revendication 3 ou 4, **caractérisé par le fait que** la section de matériau plat (18) flexible contenant du SAP s'applique contre une ou plusieurs des ouvertures d'entrée (46) ou jouxte celle(s)-ci au moins à peu près.

6. Dispositif (2) selon la revendication 5, **caractérisé par le fait que** des fluides corporels peuvent être introduits dans la section de matériau plat (18) depuis l'extérieur et transversalement à l'extension en nappe de la section de matériau plat.

7. Dispositif (2) selon une ou plusieurs des revendications précédentes, **caractérisé par le fait qu'**à l'intérieur (12) du récipient (10) est agencée au moins une autre section de matériau plat (18a, 18b).

8. Dispositif (2) selon la revendication 7, **caractérisé par le fait qu'**au moins une section de matériau plat (18a) est disposée le long de la première partie de paroi (40) et au moins une autre section de matériau plat (18b) est disposée le long de la deuxième partie de paroi (40).

9. Dispositif (2) selon une ou plusieurs des revendications précédentes, **caractérisé par le fait que** la broche ou l'entretoise (48a, 48b, 48c, 48d) s'étend dans une direction de jonction de la première et/ou de la deuxième partie de paroi (40, 42), en particulier presque jusqu'à l'autre partie de paroi (40, 42).

10. Dispositif (2) selon une ou plusieurs des revendications précédentes, **caractérisé par le fait qu'**une section de matériau plat (18) comprend une pluralité de pièces de section de matériau plat (58) disposées les unes à côté des autres dans une face.

11. Dispositif (2) selon une ou plusieurs des revendications précédentes, **caractérisé par le fait que** le récipient (10) présente une zone (50) destinée à recevoir un filtre (52) et
que ladite zone (50) destinée à recevoir le filtre (52) est délimitée ou partitionnée, au moins en partie, en particulier à la manière d'une cage, par des nervures, broches ou entretoises (54), d'une zone (56) destinée à recevoir ladite section de matériau plat (18) dans le volume intérieur (12) du récipient (10).

12. Dispositif (2) selon la revendication 11, **caractérisé par le fait qu'**une ouverture d'entrée (46) débouche dans une zone du récipient (10) opposée, de préférence diagonalement opposée, au filtre (52) agencé dans le récipient.

13. Dispositif (2) selon l'une quelconque des revendications 3 à 12, **caractérisé par le fait que** le raccord (36) communique avec l'ouverture d'entrée (46) par un canal (44) qui s'étend le long du tracé d'une paroi.

14. Dispositif (2) selon la revendication 13, **caractérisé par le fait que** le canal (44) est délimité par les première et deuxième parties de paroi (40, 42).

15. Dispositif (2) selon la revendication 13 ou 14, **caractérisé par le fait que** le canal (44) forme ou comprend une pluralité d'ouvertures d'entrée (46) dans le volume intérieur (12) du récipient (10).
